# EUROPEAN PATENT SPECIFICATION

(11) **EP 2 900 310 B1**
(45) Date of publication and mention of the grant of the patent: **03.06.2020**
(21) Application number: 13840954.5
(22) Date of filing: 24.09.2013
(51) Int. Cl.: A61M 25/08, A61M 25/095, A61M 25/09, A61M 25/00, A61M 25/01, A61M 31/00

(54) **MULTI-ANGULATED CATHETER**
MEHRWINKELIGER KATHETER
CATHÉTER À ANGLES MULTIPLES

(30) Priority: 25.09.2012 US 201261705183 P; 20.12.2012 US 201213722511
(43) Date of publication of application: 05.08.2015
(73) Proprietor: The Board Of Trustees Of The University Of Illinois, Urbana, IL 61801 (US); The United States Government as represented by The Department of Veterans Affairs, Washington, DC 20420 (US)
(72) Inventor: VIDOVICH, Mladen, I., Chicago, Illinois 60613 (US)
(74) Representative: Ricker, Mathias
(86) International application number: PCT/US2013/061301
(87) International publication number: WO 2014/052266

(56) References cited:
- WO-A1-99/30762
- DE-U1- 9 215 779
- US-A- 4 747 840
- US-A- 6 036 682
- US-A1- 2003 114 832
- US-A1- 2008 027 334
- US-A1- 2008 027 334
- US-A1- 2009 082 756
- US-B1- 6 285 903
- US-B1- 6 285 903
- US-B1- 6 926 669

## Description

### FIELD OF THE INVENTION

This invention relates generally to coronary catheters. More particularly, this invention relates to an improved, preshaped catheter for use in cardiac ventriculography.

### BACKGROUND OF THE INVENTION

Cardiac ventriculography is the radiographic examination of a ventricle of the heart that involves injection of an x-ray contrast agent, usually iodinated contrast dye, into the heart's ventricle(s), typically the left ventricle. This iodinated contrast material can be traced through the heart using special cameras or scanners thereby enabling studies of the pumping function of the heart.

However, the left ventricle may be a particularly difficult chamber of the heart to obtain x-ray imaging. Presently, so-called pigtail catheters are used, being threaded retrogradely through the aorta, around the aortic arch, and through the aortic valve until the distal tip of the catheter resides in the ventricle. Then, with the patient under an x-ray machine such as a fluoroscope, a bolus of x-ray contrast fluid is injected through the catheter at a high pressure (3.5MPa to 6.2 MPa) (500-900 psi) into the ventricle, to quickly fill the ventricle with x-ray contrast media. For a moment, details of the heart structure and action become visible by xray imaging, until the contrast media is pumped out of the ventricle.

Pigtail catheters used in cardiac ventriculography are produced by numerous medical supply companies and are available in a range of lengths and sizes to meet different needs. Currently pigtail catheters are designed as either "straight" with no angle or "angled" with angulations of 145 and 155 degrees. However, with the radial approach the angle of the aorta is not favorable for these catheters and they may be more difficult to insert into the left ventricle compared to the femoral approach. US Patent Document 6,285,903 B1 discloses an intracorporeal device having an elongated shaft.
Thus, there has developed a need for a catheter that is so dimensioned as to facilitate accessing the left ventricle from an arm of a patient. These needs are fulfilled by means of an inventive multi-angilated catheter having the features of claim 1.

### SUMMARY OF THE INVENTION

The inventor has discovered that a different angulation design, more particularly from about 105 to 140 degrees and introduction of another angle to the catheter at various distances from the tip makes the standard pigtail catheter more suitable for the radial approach.

Accordingly, this invention is a multi-angulated catheter comprising the features of claim 1. The multi-angulated catheter is flexible so as to afford being straightened when it is advanced over a guide wire. The multi-angulated catheter returns to a multi-angulated position after the guide wire is withdrawn. The first straight portion length, the first shaft length, and the first and second obtuse angles are so dimensioned as to facilitate accessing a left ventricle from an arm of a patient.

In another embodiment, this invention is a multi-angulated catheter comprising in order: (a) a coiled end; (b) a first straight portion having a first straight portion length; (c) a first shaft including a distal end connected to said first straight portion and a proximal end opposite said distal end, said first shaft having a first shaft length; and (d) a second shaft connected to said first shaft on said proximal end. The multi-angulated catheter is flexible so as to afford being straightened when it is advanced over a guide wire. The multi-angulated catheter returns to a multi-angulated position after the guide wire is withdrawn. The first shaft and the first straight portion define a first obtuse angle when viewed from a direction substantially perpendicular to a plane defined by the first and second shaft. The first straight portion extends out of plane with respect to a plane defined by the first and second shafts.

The disclosure further relates to a method for radial ventriculography comprising inserting a guide wire into an arm of a patient and inserting a multi-angulated catheter over said guide wire into said arm. The multi-angulated catheter comprises a coiled end, a first straight portion, a first shaft connected to the first straight portion at a first obtuse angle, the first shaft including a distal end connected to the first straight portion and a proximal end opposite the distal end, and a second shaft connected to the first shaft on the proximal end. The multi-angulated catheter is then advanced over the guide wire through an artery into the patient's heart. The multi-angulated catheter is flexible so as to afford being straightened when it is advanced over the guide wire. The guide wire is then withdrawn, whereupon the multi-angulated catheter returns to a multi-angulated position. The multiangulated catheter is then advanced in the multi-angulated position into a left ventricle of the patient. A radiopaque dye is then injected into the left ventricle, and a visual representation is made of the left ventricle.

Other aspects of the invention will become apparent by consideration of the detailed description and accompanying drawings.

### BRIEF DESCRIPTION OF THE FIGURES

FIG. 1. Schematic illustration of a conventional catheter.
FIG. 2. Schematic illustration of a multi-angulated catheter according to one embodiment of the invention.
FIG. 3. Schematic illustration of a multi-angulated catheter according to an alternate embodiment of the invention.

### DETAILED DESCRIPTION

Described herein is a multi-angulated catheter and methods of using the multiangulated catheter for delivering radiopaque media. The multi-angulated catheter is so dimensioned as to facilitate accessing the left ventricle from an arm of a patient. The multi-angulated catheter generally includes in order: (a) a coiled end; (b) a first straight portion having a first straight portion length; (c) a first shaft including a distal end connected to the first straight portion and a proximal end opposite the distal end, the first shaft and the first straight portion defining a first obtuse angle, the first shaft having a first shaft length; and (d) a second shaft connected to the first shaft on said proximal end. The catheter is flexible so as to afford being straightened when it is advanced over a guide wire. The catheter resiliently returns to a multi-angulated position after the guide wire is withdrawn.

### 1. Definitions

The terminology used herein is for the purpose of describing particular embodiments only and is not intended to be limiting. As used in the specification and the appended claims, the singular forms "a," "and" and "the" include plural references unless the context clearly dictates otherwise.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9, and 7.0 are explicitly contemplated.

As used herein, "radial ventriculography" refers to cardiac procedure wherein a catheter is threaded through an arm of a patient rather than the larger femoral artery in the groin. For example, the catheter may be threaded through the small radial artery in the wrist, the ulnar artery in the wrist, the brachial artery in the elbow, or any other peripheral arteries in the arm of a patient. The catheter is then advanced to the left ventricle of the patient and a radiopaque dye is then injected into the left ventricle. A visual representation is then made of the left ventricle.

### 2. Multi-Angulated Catheter

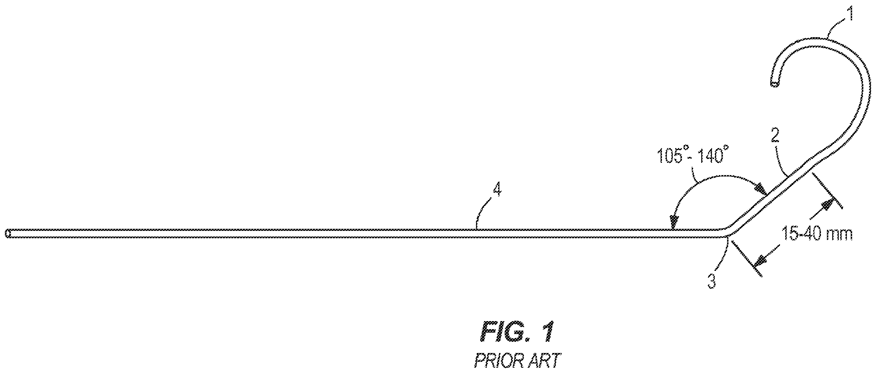

Turning now to the drawings, FIG. 1 shows a conventional catheter comprising in order: (a) a coiled end 1; (b) a first straight portion 2 having a length of about 15 to about 40 mm; (c) a first bend 3; and (d) a shaft 4, in which the first straight portion 2, the first bend 3 and the shaft 4 define an angle of about 105° to about 140°. The coiled end 1 of the catheter defines a spiral "pigtail." The tip of the pigtail comprises an open aperture and the pigtail may also contain one or more apertures to aid in uniform filling the ventricle with x-ray contrast fluid. Pigtail catheters are known in the art. See, for example US Patent No. 5,307,403. The catheter can be produced in any of the standard French sizes (4, 5, 6, 7 or 8 French).

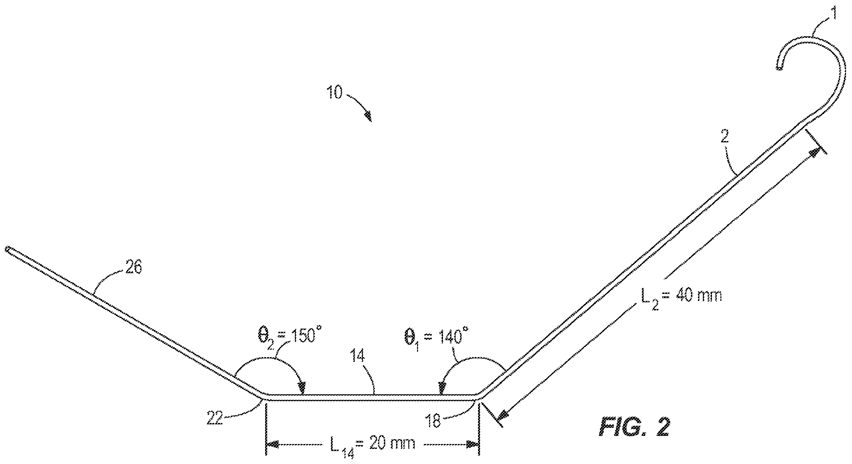

FIG. 2 shows a multi-angulated catheter 10 according to one embodiment of the invention, comprising, in order: (a) a coiled end 1; (b) a first straight portion 2 having a straight portion length L2, (c) a first shaft 14 connected to said first straight portion 2 at a first obtuse angle q 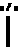 wherein said first shaft 14 has a first shaft length L14 , the first shaft 14 including a distal end 18 connected to the first straight portion 2 and a proximal end 22 opposite said distal end 18, and (d) a second shaft 26 connected to the first shaft 14 on the proximal end 22. The first shaft 14 extends from the second shaft 26 at a second obtuse angle q2. In the illustrated embodiment, the first obtuse angle qi is a positive angle, i.e., defined by a counterclockwise rotation when viewed from above, and the second obtuse angle q2 is a negative angle, i.e., defined by a clockwise rotation when viewed from above. When viewed from below, however, the first obtuse angle qi is a negative angle and the second obtuse angle q2 is a positive angle.

In an aspect, the first straight portion length L2 is so dimensioned as to facilitate accessing a left ventricle from an arm of a patient. According to the disclosure, the first straight portion length L2 is 15 mm to 40 mm. For example, FIG. 2 illustrates the first straight portion length L2 as 40 mm. In further embodiments, the first straight portion length is about 15 mm or more, about 16 mm or more, about 1 mm or more, about 18 mm or more, about 19 mm or more, about 20 mm or more, about 2 1 mm or more, about 22 mm or more, about 23 mm or more, about 24 mm or more, about 25 mm or more, about 26 mm or more, about 27 mm or more, about 28 mm or more, about 29 mm or more, about 30 mm or more, about 3 1 mm or more, about 32 mm or more, about 33 mm or more, about 34 mm or more, about 35 mm or more, about 36 mm or more, about 37 mm or more, about 38 mm or more, or about 39 mm or more. In further embodiments, the first straight portion length L2 is about 40 mm or less, about 39 mm or less, about 38 mm or less, about 37 mm or less, about 36 mm or less, about 35 mm or less, about 34 mm or less, about 33 mm or less, about 32 mm or less, about 3 1 mm or less, about 30 mm or less, about 29 mm or less, about 28 mm or less, about 27 mm or less, about 26 mm or less, about 25 mm or less, about 24 mm or less, about 23 mm or less, about 22 mm or less, about 21 mm or less, about 20 mm or less, about 19 mm or less, about 18 mm or less, about 17 mm or less, or about 16 mm or less. This includes a first straight portion length L2 of about 30 mm to about 40 mm.

In an aspect, the first shaft length L14 is so dimensioned as to facilitate accessing a left ventricle from an arm of a patient. According to the disclosure, the first shaft length L14 is 20 mm to 40 mm. For example, FIG. 2 illustrates the first shaft length L14 as 20 mm. In further embodiments, the first shaft length L14 is about 20 mm or more, about 2 1 mm or more, about 22 mm or more, about 23 mm or more, about 24 mm or more, about 25 mm or more, about 26 mm or more, about 27 mm or more, about 28 mm or more, about 29 mm or more, about 30 mm or more, about 3 1 mm or more, about 32 mm or more, about 33 mm or more, about 34 mm or more, about 35 mm or more, about 36 mm or more, about 37 mm or more, about 38 mm or more, or about 39 mm or more. In further embodiments, the first shaft length L14 is about 40 mm or less, about 39 mm or less, about 38 mm or less, about 37 mm or less, about 36 mm or less, about 35 mm or less, about 34 mm or less, about 33 mm or less, about 32 mm or less, about 3 1 mm or less, about 30 mm or less, about 29 mm or less, about 28 mm or less, about 27 mm or less, about 26 mm or less, about 25 mm or less, about 24 mm or less, about 23 mm or less, about 22 mm or less, or about 2 1 mm or less. According to the disclosure, the second shaft 26 has a second shaft length, and the second shaft length is greater than said first shaft length L14.

In an aspect, the first and second obtuse angles θ₁, θ₂ are so dimensioned as to facilitate accessing a left ventricle from an arm of a patient. In the illustrated embodiments, the first obtuse angle θ₁ is about 140°.According to the disclosure, the first obtuse angle θ₁ is 105° to 140°. In further embodiments, the first obtuse angle θ₁ is 105° or more, about 110° or more, about 115° or more, about 120° or more, about 125° or more, about 130° or more, or about 135° or more. In further embodiments, the first obtuse angle θ₁ is 140° or less, about 135° or less, about 130° or less, about 125° or less, about 120° or less, about 115° or less, or about 110° or less. For example, FIG. 2 illustrates the second obtuse angle θ₂ as 150°. In some embodiments, the second obtuse angle θ₂ is 140° or more, or about 145° or more. In further embodiments, the second obtuse angle θ₂ is 150° or less, about 145° or less, or 140°.

In some embodiments, the proximal end 22 is positioned from the coiled end 1 at a distance of about 30 mm to about 60 mm. In some embodiments, the proximal end 22 is positioned from the coiled end 1 at a distance of about 30 mm or more, about 35 mm or more, about 40 mm or more, about 45 mm or more, about 50 mm or more, or about 55 mm or more. In further embodiments, the proximal end 22 is positioned from the coiled end 1 at a distance of about 60 mm or less, about 55 mm or less, about 50 mm or less, about 45 mm or less, about 40 mm or less, or about 35 mm or less.

The multi-angulated catheter 10 is flexible so as to afford being straightened when it is advanced over a guide wire. The multi-angulated catheter 10 resiliently returns to a multi-angulated position after the guide wire is withdrawn. The catheter according to this invention may be made of any physiologically-compatible material having sufficient pliability and elasticity to permit bending of the catheter with no resultant permanent deformation. Such materials are known in the art and include, for example plastics such as polyurethane. Additionally, the catheter may further comprise, on its surface or a portion thereof a friction reducing agent which may be a hydrophilic polymer. Such agent, when wetted, as takes places in the insertion of the catheter into the aorta, provide an improved slippery surface to the catheter, which greatly assists in its placement prior to applying aliquots of x-ray contrast media to the heart. In further embodiments, the catheter optionally comprises an inner lining of resin polymer that may be braided. The inner lining may help provide a suitable stiffness to the catheter.

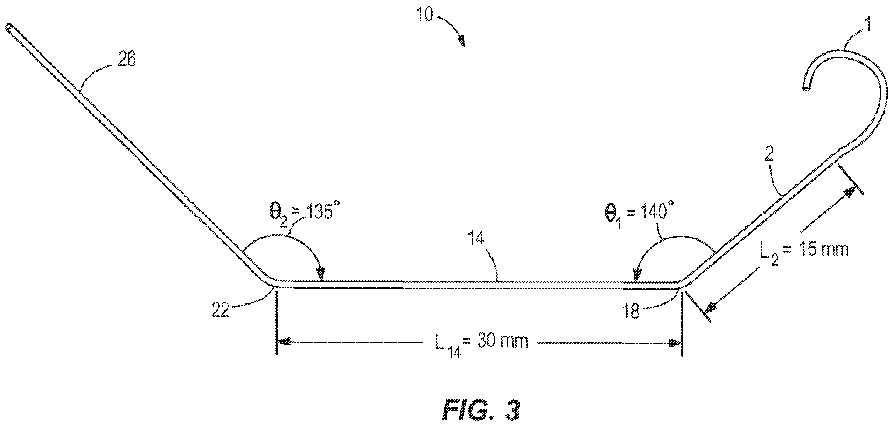

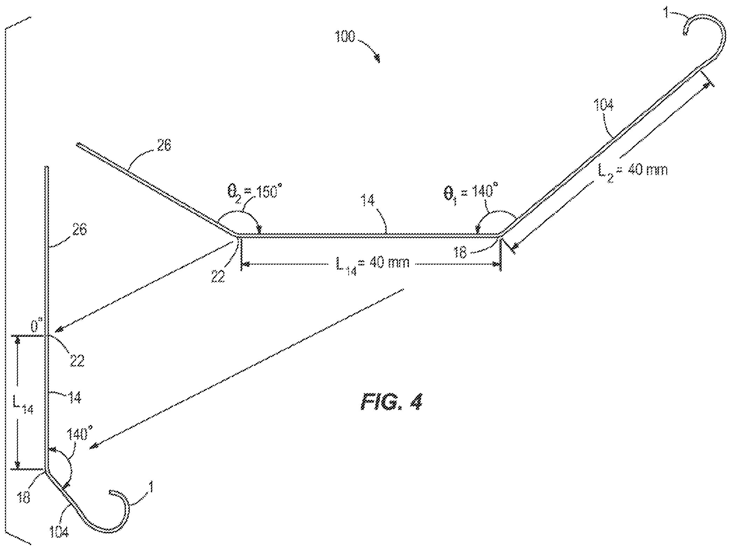

FIG. 3 shows a multi-angulated catheter according to an alternate embodiment of the invention. Like parts are identified using like reference numerals. The multi-angulated catheter 100 in this embodiment comprises in order: (a) a coiled end 1; (b) a first straight portion 104 having a first straight portion length; (c) a first shaft 14 including a distal end 18 connected to the first straight portion 104 and a proximal end 22 opposite the distal end 18, the first shaft 14 having a first shaft length L14; and (d) a second shaft 26 connected to the first shaft 14 on the proximal end 22. The multi-angulated catheter 100 is flexible so as to afford being straightened when it is advanced over a guide wire. The multi-angulated catheter 100 resiliently returns to a multi-angulated position after the guide wire is withdrawn. The first shaft 14 and the first straight portion 104 define a first obtuse angle θ₁ when viewed from a direction substantially perpendicular to a plane defined by the first and second shafts 14, 26. The first straight portion 104 extends out of plane with respect to the plane defined by the first and second shafts 14, 26.

In some embodiments, the first straight portion 104 extends from the plane defined by the first and second shafts 14, 26 at an angle of about 5° to about 140°. In some embodiments, the first straight portion 104 extends from the plane defined by the first and second shafts 14, 26 at an angle of about 5° or more, about 10° or more, about 15° or more, about 20° or more, about 25° or more, about 30° or more, about 35° or more, about 40° or more, about 45° or more, about 50° or more, about 55° or more, about 60° or more, about 65° or more, about 70° or more, about 75° or more, about 80° or more, about 85° or more, about 90° or more, about 95° or more, about 100° or more, about 105° or more, about 110° or more, about 115° or more, about 120° or more, about 125° or more, about 130° or more, or about 135° or more. In further embodiments, the first straight portion 104 extends from the plane defined by the first and second shafts 14, 26 at an angle of about 140° or less, about 135° or less, about 130° or less, about 125° or less, about 120° or less, about 115° or less, about 110° or less, about 105° or less, about 100° or less, about 95° or less, about 90° or less, about 85° or less, about 80° or less, about 75° or less, about 70° or less, about 65° or less, about 60° or less, about 55° or less, about 50° or less, about 45° or less, about 40° or less, about 35° or less, about 30° or less, about 25° or less, about 20° or less, about 15° or less, or about 10° or less. The first straight portion 104 in this embodiment creates a three-dimensional profile for the multi-angulated catheter 100. The three-dimensional profile of the multi-angulated catheter 100 can facilitate accessing a left ventricle from an arm of a patient.

### 3. Method of Using the Multi-Angulated Catheter

The present disclosure is also directed to a method of using the multi-angulated catheter. In an operation such as in a radial ventriculography, a physician inserts a guide wire into an arm of a patient. The guide wire may be a conventional J-tip wire with a diameter of 0.89 mm or 0.97 mm. The multi-angulated catheter 10, 100 is then inserted over the guide wire into the arm and advanced over the guide wire through an artery into the patient's heart. The multi-angulated catheter 10, 100 is flexible so as to afford being straightened when it is advanced over the guide wire. In some embodiments, the multi-angulated catheter 10, 100 is advanced through the axillary artery, the subclavian artery, the innominate artery, and the ascending aorta. The guide wire is then withdrawn, whereupon the multi-angulated catheter 10, 100 resiliently returns to a multi-angulated position.

The multi-angulated catheter 10, 100 is then advanced in the multi-angulated position into a left ventricle of the patient. In some examples, the multi-angulated catheter is advanced in a clockwise fashion into the left coronary cusp, which may cause the multi-angulated catheter 10, 100 to curl upwards. In the upwardly curled position, the multiangulated catheter 10, 100 may be withdrawn slightly and rotated slightly counterclockwise, so as to allow it to prolapse in the left ventricular cavity.

A radiopaque dye is then injected into the left ventricle, and a visual representation is made of the left ventricle. In some examples, the position of the multiangulated catheter 10, 100 may be adjusted before or during the dye injection and visual representation. For example, the position of the multi-angulated catheter 10, 100 may be adjusted with slight advancements such as forward and backward, and/or clockwise and counterclockwise, to find a central ventricular position. Such adjustments may help avoiding myocardial staining and ventricular ectopy, and may also help achieving full cavity opacification. In some examples, pressure measurements may be obtained when the multi-angulated catheter 10, 100 is positioned in the aorta or in the ventricle.

After the ventriculography is performed, the multi-angulated catheter 10, 100 is withdrawn into the ascending aorta. The guide wire is then inserted to straighten the multiangulated catheter 10, 100, and the catheter 10, 100 is then removed from the patient over the guide wire.

The foregoing has been provided for illustrative purposes only and is not intended to limit the scope of the invention as set forth in the claims.

## Claims

1. A multi-angulated catheter (10; 100) comprising, in order:
(a) a coiled end (1);
(b) a first straight portion (2; 104) having a first straight portion length, wherein said first straight portion length is 15 mm to 40 mm;
(c) a first shaft (14) connected to said first straight portion (2; 104) at a first obtuse angle, wherein said first obtuse angle is 105° to 140°, wherein said first shaft (14) has a first shaft length, wherein said first shaft length is 20 mm to 40 mm, said first shaft (14) including a distal end (18) connected to said first straight portion (2; 104) and a proximal end (22) opposite said distal end (18);
(d) a second shaft (26) connected to said first shaft (14) on said proximal end (22), said first shaft (14) extending from said second shaft (26) at a second obtuse angle, one of said first and second obtuse angles being a positive angle and the other being a negative angle, wherein said multi-angulated catheter (10; 100) is flexible so as to afford being straightened when it is advanced over a guide wire, said multi-angulated catheter (10; 100) returning to a multi-angulated position after said guide wire is withdrawn, and wherein said first straight portion length, said first shaft length, and said first and second obtuse angles are so dimensioned as to facilitate accessing a left ventricle from an arm of a patient; and
wherein said second shaft (26) has a second shaft length, and said second shaft length is greater than said first shaft length; and
wherein said second obtuse angle is 140° to 150°.

2. The multi-angulated catheter (10; 100) of claim 1, wherein said first straight portion length is 15 mm to 25 mm.

3. The multi-angulated catheter (10; 100) of claim 1, wherein said first straight portion length is 30 mm to 40 mm.

4. The multi-angulated catheter (10; 100) of any one of claims 1-3, wherein said first obtuse angle is 125° to 140°.

5. The multi-angulated catheter (10; 100) of any one of claims 1-4, wherein said first shaft length is 30 mm to 40 mm.

6. The multi-angulated catheter (10; 100) of any one of claims 1-5, wherein said proximal end (22) is positioned from said coiled end (1) at a distance of 30 mm to 60 mm.

7. The multi-angulated catheter (10; 100) of any one of claims 1-6, wherein said coiled end (1) comprises an open aperture in a tip of said coiled end (1).

8. The multi-angulated catheter (10; 100) of any one of claims 1-7, wherein said coiled end (1) comprises one or more apertures in a sidewall of said coiled end (1).

9. The multi-angulated catheter (10; 100) of any one of claims 1-8, wherein said coiled end (1) comprises a pigtail loop.

10. The multi-angulated catheter (10; 100) of any one of claims 1-9, comprising an external diameter of 3 French to 8 French.

11. The multi-angulated catheter (10; 100) of any one of claims 1-10, wherein the first straight portion (2; 104) extends out of a plane defined by the first and second shafts (14, 26).

12. The multi-angulated catheter (10; 100) of claim 11, wherein the first straight portion (2; 104) extends out of the plane at an angle between 5° and 140°.

13. The multi-angulated catheter (10; 100) of any one of claims 1-12, further comprising a friction reducing agent disposed on a portion of a surface of the multi-angulated catheter (10; 100).

## Patentansprüche

1. Ein mehrfach abgewinkelter Katheter (10; 100), in Reihenfolge aufweisend:
(a) ein eingerolltes Ende (1);
(b) einen ersten geraden Abschnitt (2; 104) mit einer ersten geraden Abschnittslänge, wobei die erste gerade Abschnittslänge 15 mm bis 40 mm beträgt;
(c) einen ersten Schaft (14), der mit dem ersten geraden Abschnitt (2; 104) unter einem ersten stumpfen Winkel verbunden ist, wobei der erste stumpfe Winkel 105° bis 140° beträgt, wobei der erste Schaft (14) eine erste Schaftlänge aufweist, wobei die erste Schaftlänge 20 mm bis 40 mm beträgt, der erste Schaft (14) ein mit dem ersten geraden Abschnitt (2; 104) verbundenes distales Ende (18) beinhaltet, sowie ein gegenüber dem distalen Ende (18) proximales Ende (22);
(d) einen zweiten Schaft (26), der mit dem ersten Schaft (14) an dem proximalen Ende (22) verbunden ist, wobei sich der erste Schaft (14) von dem zweiten Schaft (26) unter einem zweiten stumpfen Winkel erstreckt, wobei einer der ersten und zweiten stumpfen Winkel ein positiver Winkel und der andere ein negativer Winkel ist, wobei der mehrfach gewinkelte Katheter (10; 100) flexibel ist, so dass er sich gerade ausrichten lässt, wenn er über einen Führungsdraht geschoben wird, wobei der mehrfach gewinkelte Katheter (10; 100) in eine mehrfach gewinkelte Position zurückkehrt, nachdem der Führungsdraht herausgezogen ist, und wobei die erste gerade Abschnittslänge, die erste Schaftlänge und der erste und zweite stumpfe Winkel so dimensioniert sind, dass sie den Zugang zu einem linken Ventrikel von einem Arm eines Patienten aus erleichtern; und
wobei der zweite Schaft (26) eine zweite Schaftlänge aufweist und die zweite Schaftlänge größer als die erste Schaftlänge ist; und
wobei der zweite stumpfe Winkel 140° bis 150° beträgt.

2. Mehrfach abgewinkelter Katheter (10; 100) nach Anspruch 1, wobei die erste gerade Abschnittslänge 15 mm bis 25 mm beträgt.

3. Mehrfach abgewinkelter Katheter (10; 100) nach Anspruch 1, wobei die erste gerade Abschnittslänge 30 mm bis 40 mm beträgt.

4. Mehrfach abgewinkelter Katheter (10; 100) nach einem der Ansprüche 1-3, wobei der erste stumpfe Winkel 125° bis 140° beträgt.

5. Mehrfach abgewinkelter Katheter (10; 100) nach einem der Ansprüche 1-4, wobei die erste Schaftlänge 30 mm bis 40 mm beträgt.

6. Mehrfach abgewinkelter Katheter (10; 100) nach einem der Ansprüche 1-5, wobei das proximale Ende (22) in einem Abstand von 30 mm bis 60 mm von dem eingerollten Ende (1) positioniert ist.

7. Mehrfach abgewinkelter Katheter (10; 100) nach einem der Ansprüche 1-6, wobei das eingerollte Ende (1) eine offene Apertur in einer Spitze des eingerollten Endes (1) aufweist.

8. Mehrfach abgewinkelter Katheter (10; 100) nach einem der Ansprüche 1-7, wobei das eingerollte Ende (1) eine oder mehrere Aperturen in einer Seitenwand des eingerollten Endes (1) aufweist.

9. Mehrfach abgewinkelter Katheter (10; 100) nach einem der Ansprüche 1-8, wobei das eingerollte Ende (1) eine Pigtailschleife aufweist.

10. Mehrfach abgewinkelter Katheter (10; 100) nach einem der Ansprüche 1-9 aufweisend einem Außendurchmesser von 3 French bis 8 French.

11. Mehrfach abgewinkelter Katheter (10; 100) nach einem der Ansprüche 1-10, wobei sich der erste gerade Abschnitt (2; 104) aus einer durch den ersten und zweiten Schaft (14, 26) definierten Ebene heraus erstreckt.

12. Mehrfach abgewinkelter Katheter (10; 100) nach Anspruch 11, wobei der erste gerade Abschnitt (2; 104) sich in einem Winkel zwischen 5° und 140° aus der Ebene heraus erstreckt.

13. Mehrfach abgewinkelter Katheter (10; 100) nach einem der Ansprüche 1-12, ferner ein reibungsverminderndes Mittel aufweisend, dass auf einem Teil einer Oberfläche des mehrfach abgewinkelten Katheters (10; 100) angeordnet ist.

## Revendications

1. Cathéter à angles multiples (10 ; 100) comprenant, dans cet ordre :
(a) une extrémité enroulée (1) ;
(b) une première partie droite (2 ; 104) ayant une première longueur de partie droite, dans lequel ladite première longueur de partie droite est de 15 mm à 40 mm ;
(c) une première tige (14) reliée à ladite première partie droite (2 ; 104) selon un premier angle obtus, dans lequel ledit premier angle obtus est de 105° à 140°, dans lequel ladite première tige (14) présente une première longueur de tige, dans lequel ladite première longueur de tige est de 20 mm à 40 mm, ladite première tige (14) incluant une extrémité distale (18) reliée à ladite première partie droite (2 ; 104) et une extrémité proximale (22) à l'opposé de ladite extrémité distale (18) ;
(d) une deuxième tige (26) reliée à ladite première tige (14) au niveau de ladite extrémité proximale (22), ladite première tige (14) s'étendant depuis ladite deuxième tige (26) selon un deuxième angle obtus, un parmi lesdits premier et deuxième angles obtus étant un angle positif et l'autre étant un angle négatif, dans lequel ledit cathéter à angles multiples (10 ; 100) est souple de manière à pouvoir être redressé lorsqu'il est avancé le long d'un fil-guide, ledit cathéter à angles multiples (10 ; 100) revenant à une position à angles multiples après que ledit fil-guide est retiré, et dans lequel ladite première longueur de partie droite, ladite première longueur de tige et lesdits premier et deuxième angles obtus sont dimensionnés de sorte à faciliter l'accès à un ventricule gauche depuis un bras d'un patient ; et
dans lequel ladite deuxième tige (26) possède une deuxième longueur de tige, et ladite deuxième longueur de tige est supérieure à ladite première longueur de tige ; et
dans lequel ledit deuxième angle obtus est de 140° à 150°.

2. Cathéter à angles multiples (10 ; 100) selon la revendication 1, dans lequel ladite première longueur de partie droite est de 15 mm à 25 mm.

3. Cathéter à angles multiples (10 ; 100) selon la revendication 1, dans lequel ladite première longueur de partie droite est de 30 mm à 40 mm.

4. Cathéter à angles multiples (10 ; 100) selon l'une quelconque des revendications 1-3, dans lequel ledit premier angle obtus est de 125° à 140°.

5. Cathéter à angles multiples (10 ; 100) selon l'une quelconque des revendications 1-4, dans lequel ladite première longueur de tige est de 30 mm à 40 mm.

6. Cathéter à angles multiples (10 ; 100) selon l'une quelconque des revendications 1-5, dans lequel ladite extrémité proximale (22) est positionnée depuis ladite extrémité enroulée (1) à une distance de 30 mm à 60 mm.

7. Cathéter à angles multiples (10 ; 100) selon l'une quelconque des revendications 1-6, dans lequel ladite extrémité enroulée (1) comprend un orifice ouvert sur une pointe de ladite extrémité enroulée (1).

8. Cathéter à angles multiples (10 ; 100) selon l'une quelconque des revendications 1-7, dans lequel ladite extrémité enroulée (1) comprend un ou plusieurs orifices dans une paroi de ladite extrémité enroulée (1).

9. Cathéter à angles multiples (10 ; 100) selon l'une quelconque des revendications 1-8, dans lequel ladite extrémité enroulée (1) comprend une boucle en queue de cochon.

10. Cathéter à angles multiples (10 ; 100) selon l'une quelconque des revendications 1-9, comprenant un diamètre externe de 3 French à 8 French.

11. Cathéter à angles multiples (10 ; 100) selon l'une quelconque des revendications 1-10, dans lequel la première partie droite (2 ; 104) s'étend hors d'un plan défini par les première et deuxième tiges (14, 26).

12. Cathéter à angles multiples (10 ; 100) selon la revendication 11, dans lequel la première partie droite (2 ;104) s'étend hors du plan selon un angle entre 5° et 140°.

13. Cathéter à angles multiples (10 ; 100) selon l'une quelconque des revendications 1-12, comprenant en outre un agent de réduction de frottement disposé sur une partie d'une surface du cathéter à angles multiples (10 ; 100).
